# EUROPÄISCHE PATENTSCHRIFT

(11) **EP 0 877 767 B1**
(45) Veröffentlichungstag und Bekanntmachung des Hinweises auf die Patenterteilung: **21.07.1999**
(21) Anmeldenummer: 97901556.7
(22) Anmeldetag: 20.01.1997
(51) Int. Cl.: C08G 18/18, B27N 3/00, C08L 97/02

(54) **VERFAHREN ZUR HERSTELLUNG VON PRESSWERKSTOFFEN MIT POLYISOCYANAT-BINDEMITTELN UNTER MITVERWENDUNG VON LATENTEN, WÄRMEAKTIVIERBAREN KATALYSATOREN**
METHOD OF PRODUCING PRESS-MOULDING MATERIALS WITH POLYISOCYANATE BINDERS AND USING LATENT, HEAT-ACTIVABLE CATALYSTS
PROCEDE DE PRODUCTION DE MATERIAUX MOULES PAR COMPRESSION AVEC DES LIANTS POLYISOCYANATE, AVEC UTILISATION SIMULTANEE DE CATALYSEURS THERMO-ACTIVABLES LATENTS

(30) Priorität: 31.01.1996 DE 19603330
(43) Veröffentlichungstag der Anmeldung: 18.11.1998
(73) Patentinhaber: BAYER AG, 51368 Leverkusen (DE)
(72) Erfinder: HAAS, Peter, D-42781 Haan (DE); VEHLEWALD, Peter, D-42799 Leichlingen (DE); KASPEREK, Peter, D-53804 Much (DE)
(86) Internationale Anmeldenummer: EP9700237
(87) Internationale Veröffentlichungsnummer: WO9728202

(56) Entgegenhaltungen:
- EP-A- 0 133 680
- DE-A- 4 229 396
- US-A- 4 186 255
- US-A- 4 632 785

## Beschreibung

Es wird ein Verfahren zur Herstellung von Preßwerkstoffen, vorzugsweise Spanplatten, durch Heißverpressen von mit Polyisocyanaten als Bindemittel vermischten und/oder imprägnierten lignosecellulosehaltigen Rohstoffen unter Mitverwendung von neuartigen, latenten, durch Wärme aktivierbaren Katalysatoren auf der Basis von Ammoniumsalzen, wie sie durch Umsetzung von primären, sekundären und/oder tertiären Aminen mit Malonsäure entstehen, beansprucht.

Preßwerkstoffe, wie z.B. Spanplatten, Verbundplatten oder andere Formkörper werden gewöhnlich so hergestellt, daß man das anorganische oder organische Rohmaterial, z.B. eine Masse aus Holzschnitzeln, Holzfasern und/oder anderen lignosecellulosehaltigem Material, mit Polyisocyanaten und Wasser, gegebenenfalls Polyolen oder anderen Bindemitteln wie Harnstoff/Formaldehyd- oder Phenol/Formaldehyd-Harzen, als sogenannte Mischverleimung heiß verpreßt. Die Verwendung von Polyisocyanaten als Bindemittel verbessert die Stabilität und das Feuchteverhalten der Produkte und erhöht deren mechanische Eigenschaften. Darüber hinaus besitzen Polyisocyanate als Bindemittel, wie z.B. in der DE-OS 2 109 686 offenbart, weitreichende verfahrenstechnische Vorteile.

Prinzipiell können bei den Verfahren des Standes der Technik (siehe z.B. DE-AS 2 711 958) auch Katalysatoren der aus der Polyurethanchemie an sich bekannten Art, z.B. solche, wie sie in der DE-OS 2 854 384 auf den Seiten 26 bis 29 und 31 bis 33 genannt sind, mitverwendet werden, um Preßzeiten zu verkürzen. Dies ist bei sogenannten Einetagenpressen von besonderer Wichtigkeit. Hierbei kommt es jedoch meist bereits beim Vermischen der Komponenten und der Lagerung der mit Isocyanaten beleimten Späne vor der Verpressung zu unerwünschter Schaumbildung und vorzeitiger Abbindung infolge der sofort einsetzenden Katalysatorwirkung auf die reaktiven NCO-Gruppen. Deswegen muß man meist auf die Mitverwendung von Katalysatoren verzichten und längere Preßzeiten in Kauf nehmen.

Aus der EP 133 680 ist ein Verfahren zur Herstellung von Preßwerkstoffen unter dem Einsatz von Polyurethanen als Bindemittel bekannt, bei dem man als wärmeaktivierbare Katalysatoren tertiäre oder quartäre Ammoniumphosphonate oder quartäre Ammoniumphosphate einsetzt. Auch die bei Verwendung dieser Katalysatoren zu beobachtende Verkürzung der Preßzeit ist für eine wirtschaftliche Verfahrensdurchführung immer noch nicht ausreichend. Auch mit den in der DE 4 229 396 und DE 3 438 735 beschriebenen Verfahren zur Herstellung von Span- oder Faserplatten unter Verwendung von Polyisocyanaten als Bindemittel sind noch keine ausreichend kurzen Preßzeiten erreichbar.

Aufgabe der vorliegenden Erfindung ist es daher, ein latentes Katalysatorsystem für die Herstellung von Preßwerkstoffen mit Polyisocyanat-Bindemitteln zur Verfügung zu stellen, das Reaktionen von Polyisocyanaten bei erhöhten Temperaturen aktivieren kann, aber bis zu Temperaturen von 80°C sich noch nicht katalytisch wirkend verhält.

Dieses Katalysatorsystem kann bereits dem verwendeten Polyisocyanat oder der zur Herstellung von Preßkörpern benutzen Formulierung aus Polyisocyanaten, Wasser, lignosecellulosehaltigen Materialien wie Fasern, Spänen oder strohartigen Fasern und evtl. anderen mitzuverwendenden Polyhydroxylverbindungen hinzugemischt werden, ohne daß es bei Raumtemperatur und beim Vermischen und Lagern bei Temperaturen bis zu 80°C zu unerwünschten Reaktionen kommt. Die Preßzeiten können andererseits infolge der bei der höheren Temperatur bewirkten Katalyse durch schnelleres Abbinden bei der Pressung erheblich gesenkt werden. Überraschenderweise wurde nun gefunden, daß die nachstehend näher beschriebenen Katalysatoren auf der Basis von Aminsalzen der Malonsäure für die beschriebene Aufgabe sehr gut geeignet sind, da sie bis 80°C latente Katalysatoren in der Polyisocyanat-Formulierung darstellen, aber bei den Preßtemperaturen die Isocyanatreaktionen katalysieren und somit die oben gestellten Bedingungen erfüllen.

Gegenstand der Erfindung ist ein Verfahren zur Herstellung von Preßwerkstoffen, vorzugsweise von Holzspanplatten, durch Heißverpressen von mit Polyisocyanaten als Bindemittel vermischten und/oder imprägnierten lignocellulosehaltigen Werkstoffen unter Verwendung durch Wärme aktivierbare Katalysatoren, dadurch gekennzeichnet, daß als Katalysatoren Ammoniumsalze aus der Umsetzung von Aminen mit Malonsäure eingesetzt werden.

Bei den erfindungsgemäß einzusetzenden Ammoniumsalzen handelt es sich bevorzugt um solche, die durch Umsetzung von mono- oder polyfunktionellen, primären, sekundären und/oder tertiären Aminen mit Malonsäure erhalten werden können.

Als Aminkomponente der erfindungsgemäß einzusetzenden Ammoniumsalze können dabei alle Amine eingesetzt werden, wie sie z.B. in der EP 133 680 genannt werden. Besonders bevorzugt sind dabei die tertiären Amine, insbesondere N,N-Dimethylaminoethanol, Dimethylaminopropylharnstoff, Bis-2,2'-Dimethylaminoethylether, N-Methylimidazol und N-Methyl-2-aranorboman.

Die erfindungsgemäß zu verwendenden Ammoniumsalze können durch Umsetzung der beispielhaft genannten Amine mit Malonsäure erhalten werden.

Die Herstellung von Ammoniumsalzen der Malonsäure ist dem Fachmann prinzipiell bekannt. Üblicherweise werden dafür die Amine mit in Wasser gelöst und anschließend mit Malonsäure neutralisiert, wobei vorzugsweise äquimolare Mengen an Amin und Malonsäure eingesetzt werden.

Die überraschende Latenzwirkung bei 60-80°C und die Aktivierungswirkung ab 100°C wird dadurch noch unterstrichen, daß die Malonsäure in der Reihe der Dicarbonsäuren an sich die thermolabilste dieser Säuren darstellt, also im Prinzip ein gegenteiliges Verhalten bezüglich der Latenzphase zu erwarten ist.

Dies gilt nicht nur für die in äquivalenten Mengen neutralisierten Ammoniumsalze der Malonsäure, sondern auch für Produkte, die sogar in unterschüßigen Mengen neutralisiert werden und demnach in saurer, also teil neutralisierter Form, vorliegen.

Die Umsetzung der Malonsäure zu den erfindungsgemäßen Ammoniummalonaten kann in Wasser oder aber genau so vorteilhaft in Lösemitteln erfolgen, die gegenüber Isocyanaten inert sind, wie z.B. Dimethylacetamid, N-Methylpyrrolidon, N-Methylcaprolactam, N,N'-Dimethylimidazolidon. Diese Solventien erlauben eine Dosierung der latenten Aktivatoren in den Isocyanaten, die auf die Holzwerkstoffe versprüht werden.

Bei den erfindungsgemäß zu verwendenden Katalysatoren handelt es sich im allgemeinen um farblose Lösungen in Wasser oder organischen Solventien, welche die Ammoniumsalze der Malonsäure enthalten. Bei Temperaturen von unterhalb 80°C sind die erfindungsgemäßen Katalysatoren ohne nennenswerte katalytische Aktivität bezüglich der Isocyanatadditionsreaktion. Erst bei Temperaturen oberhalb 90°C, insbesondere innerhalb des Temperaturbereichs von 90-150°C, vorzugsweise 90-110°C, kommt es zu einer ausgeprägten katalytischen Wirkung der erfindungsgemäß eingesetzten Katalysatoren. Hierdurch wird erreicht, daß einerseits die Lagerstabilität der die erfindungsgemäßen Katalysatoren enthaltenden Reaktionsgemischen aus Polyisocyanaten und Rohwerkstoffen bei den genannten Temperaturen nur wenig unter der Lagerstabilität entsprechender, unkatalysieter Reaktionsgemische liegt, während andererseits bei höheren Temperaturen innerhalb der genannten Bereiche die erwünschte, starke Beschleunigung der Isocyanat-Additionsreaktion erfolgt. In vorteilhafter Weise kommt es somit zu einer Verkürzung der Preßzeit.

Die erfindungsgemäßen Katalysatoren können selbstverständlich auch in modifizierten Polyisocyanat-Bindemitteln gelöst sein, so z.B. in wäßriger Polyisocyanat-Emulsionen, wie sie unter Zusatz von Emulgatoren wie z.B. Polyethylenglykolen, Leim, Polyvinylpyrrolidon, Polyacrylamiden, welche gegebenenfalls noch Polyethylendispersionen und Holzschutzmittel enthalten, oder in solchen modifizierten wässrigen Polyisocyanaten-Emulsionen, welche durch Modifizierung mit monofunktionellen Polyethylenoxidderivaten oder durch Zusatz von Phosphor- bzw. Sulfonsäuren hydrophiliert sind.

Als Isocyanatkomponente kommen bei der Durchführung des erfindungsgemäßen Verfahrens aliphatische, cycloaliphatische, araliphatische, aromatische und heterocyclische Polyisocyanate in Betracht, wie sie z.B. von W. Siefken in Justus Liebigs Annalen der Chemie, 562, Seiten 75 bis 136, beschrieben werden, beispielsweise solche der Formel

Q(NCO)ₙ

in der
- n: 2 bis 4, vorzugsweise 2, und
- Q: einen aliphatischen Kohlenwasserstoffrest mit 2 bis 18, vorzugsweise 6 bis 10 C-Atomen, einen cycloaliphatischen Kohlenwasserstoffrest mit 4 bis 23, vorzugsweise 5 bis 13 C-Atomen, einen aromatischen Kohlenwasserstoffrest mit 6 bis 23, vorzugsweise 6 bis 13 C-Atomen, oder einen araliphatischen Kohlenwasserstoffrest mit 8 bis 15, vorzugsweise 8 bis 13 C-Atomen,
bedeuten, z.B. 4,4'-Diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat sowie beliebige Gemische dieser Isomeren oder deren polymere Typen dieser Reihe.

Bevorzugt werden in der Regel die technisch leicht zugänglichen Polyisocyanate, zB. das 2,4- und 2,6-Toluylendiisocyanat sowie beliebige Gemische dieser Isomeren ("TDI"), sowie besonders bevorzugt Polyphenyl-polymethylen-polyisocyanate, wie sie durch Anilin-Form-aldehyd-Kondensation und anschließende Phosgenierung hergestellt werden ("rohes MDI"). Gegebenenfalls können die eingesetzten Polyisocyanate auch modifiziert sein. Besonders bevorzugt setzt man als Polyisocyanatkomponente höherkernige Isocyanate der Phenylmethandiisocyanat-Reihe (PMDI-Typen) ein.

Für die Durchführung des erfindungsgemäßen Verfahrens können auch endständige Isocyanatgruppen aufweisende Prepolymere mit einem mittleren Molekulargewicht von ca. 300 bis 2000 eingesetzt werden, wie sie durch Umsetzung von höhermolekularen und/oder niedermolekularen Polyolen mit einem Überschuß an Polyisocyanat in an sich bekannter Weise erhalten werden.

Als Polyole können alle in der Polyurethanchemie gebräuchlichen höhermolekularen Polyole, insbesondere zwei bis acht Hydroxylgruppen aufweisende Verbindungen, speziell solche der Molmassen 400 bis 10 000, vorzugsweise 600 bis 5 000, z.B. mindestens zwei, in der Regel zwei bis acht, vorzugsweise aber zwei bis vier, Hydroxylgruppen aufweisende Polyester, Polyether, Polythioether, Polyacetale, Polycarbonate und Polyesteramide, wie sie beispielsweise für die Herstellung von homogenen und von zellförmigen Polyurethanen an sich bekannt sind, eingesetzt werden.

Geeignete lignocellulosehaltige Rohstoffe, die mit der erfindungsgemäßen Polyisocyanat-Aktivator-Formulierung gebunden werden können, sind beispielsweise Holz, Rinde, Kork, Bagasse, Stroh, Flachs, Bambus, Alfagras, Reisschalen, Sisal- und Kokosfasern. Das Material kann dabei in Form von Granulaten, Spänen, Fasern oder Mehl vorliegen und einen Wassergehalt von z.B. 0 bis 35 Gew.-%, vorzugsweise von 5 bis 25 Gew.-%, aufweisen. Es wird mit dem Bindemittel in einer Menge von 1 bis 100, vorzugsweise 2 bis 12 Gew.-%, versetzt und - im allgemeinen unter Einwirkung von Druck und Hitze - zu Platten oder Formkörpern verpreßt.

Bezogen auf das Polyisocyanat-Bindemittel werden 0,1 bis 20, bevorzugt 0,1 bis 15 Gew.-% erfindungsgemäßer Katalysator zugesetzt.

Selbstverständlich können erfindungsgemäß jedoch auch aus anderen organischen (z.B. Kunststoffabfälle aller Art) und/oder anorganischen Rohstoffen (z.B. Blähglimmer oder Silikatkugeln) Preßkörper hergestellt werden.

Bei der erfindungsgemäßen Verwendung wird das zu verpressende Material mit dem Bindemittel vermischt, und zwar zweckmäßig durch Besprühen mit dem erfindungsgemäßen Bindemittel, um so eine möglichst homogene Verteilung zu erreichen.

In der Praxis können Verzögerungsperioden zwischen den einzelnen Verfahrensstufen (Herstellung der Formulierung; Besprühen des zu verwendenden Materials) auftreten, sowie auch Verzögerungen in Folge von Arbeitsfehlern oder in Folge einer Neueinstellung der Verfahrensbedingungen. In wünschenswerter Weise verläuft jedoch die Beschleunigung der Isocyanatreaktionen der entsprechend beleimten Holzwerkstoffe durch die erfindungsgemäßen Katalysatoren bei Temperaturen bis 80°C genügend langsam, so daß eine Verzögerung von mindestens 2 Stunden bis zu mehreren Stunden besonders bei niedrigen Temperaturen zwischen Herstellung der Isocyanat-Aktivator-Formulierung und dem Heißpressen in Kauf genommen werden kann. Die Reaktionsgeschwindigkeit kann noch verlangsamt werden, indem die Reaktivität der Polyisocyanate verändert wird. So kann man beispielsweise durch einen erhöhten Anteil an 2,4'- und/oder 2,2'-Diphenylmethandiisocyanat (in Relation zum 4,4'-Isomeren) die Reaktionsgeschwindigkeit deutlich herabsetzen.

In analoger Weise können auch mehrlagige Platten oder Formteile aus Furnieren, Papieren oder Geweben hergestellt werden. Auch mehrschichtige Platten oder Formteile aus Furnieren und Streifen-, Stab- oder Stäbchen-Mittellagen, sogenannte Tischlerplatten, können erfindungsgemäß hergestellt werden, indem man die Furniere wie oben beschrieben mit der Isocyanat-Aktivator-Formulierung behandelt und anschließend mit den Mittel lagen - in der Regel bei erhöhter Temperatur und erhöhtem Druck - verpreßt. Vorzugsweise werden dabei Temperaturen von 80 bis 250°C, besonders bevorzugt 100 bis 220°C, eingehalten. Der Anfangspreßdruck liegt auch hier vorzugsweise zwischen 5 und 150 bar; im Laufe des Preßvorganges fällt dann der Druck meist bis gegen 0 ab.

Erfindungsgemäß können die Polyisocyanat-Aktivator-Formulierungen auch in Kombination mit den oben beschriebenen Polyhydroxylverbindungen in einem NCO/OH-Verhältnis von 1,1:1 bis 10:1, vorzugsweise 1,5:1 bis 5:1, eingesetzt werden. Es ist dabei möglich, die beiden Komponenten getrennt oder als reaktives Gemisch einzusetzen. Praktische Bedeutung haben derartige Kombinationen von Polyisocyanat und Polyhydroxylverbindungen als Bindemittel z.B. bei der Bindung von Korkschrot. Es ist auch möglich, an sich bekannte Treibmittel in einer Menge von ca. 0,5 bis 30 Gew.-%, bezogen auf Binde- oder Imprägniermittel und/oder andere die Schaumbildung oder die chemische Reaktion zwischen Polyisocyanaten, lignocellulosehaltigem Material und gegebenenfalls Polyhydroxylverbindungen beeinflussende Additive wie Stabilisatoren, in einer Menge von 0,05 bis 10 Gew.-%, bezogen auf Bindemittel- bzw. Imprägniermittel, zuzusetzen.

Die erfindungsgemäß als Bindemittel zu verwendenden Polyisocyanat-Aktivator-Formulierungen können auch mit den in der Holzwerkstoffindustrie bisher überwiegend eingesetzten wäßrigen Lösungen von Kondensationsprodukten aus Formaldehyd mit Harnstoff und/oder Melamin und/oder Phenol kombiniert werden (Mischverleimung), aber auch mit anderen, bisher weniger üblichen Binde- und Imprägniermitteln, wie z.B. Sulfitablauge (Lignin-Sulfonat oder anderen technischen Ligninlösungen des Holzaufschlusses) oder gerbstoffartigen Verbindungen wie Tannin, wo z.B. ein Mischungsverhältnis der erfindungsgemäßen mit diesen zusätzlichen Bindemitteln zwischen 1:10 und 10:1, vorzugsweise zwischen 1:5 und 5:1, eingehalten werden kann und wobei man die erfindungsgemäßen Bindemittel und die zusätzlichen Bindemittel entweder separat oder auch in Mischung einsetzen kann.

Besonders vorteilhaft sind derartige Kombinationen bei der Herstellung von mehrschichtigen Platten mit speziellen Eigenschaften. Man kann z.B. die äußeren Schichten mit konventionellen Klebstoffen (allein oder gemeinsam mit dem Polyisocyanat-Bindemittel und eine oder mehrere innere Schichten mit dem erfindungsgemäß zu verwendenden Polyisocyanat-Bindemittel (allein oder gemeinsam mit konventionellen Klebstoffen) versetzen und anschließend miteinander verpressen.

Bei der Herstellung von Spanplatten, besonders mehrschichtiger Spanplatten, ergibt sich oft das Problem, die Späne auch in den Mittel schichten der Spanplatten bei möglichst kurzen Preßzeiten noch vollständig mit den Polyisocyanat-Bindemitteln umzusetzen. Hier wirkt sich der Vorteil der erfindungsgemäßen, latent wirksamen wärmeaktivierbaren Katalysatoren besonders günstig aus, indem gerade in den Mittelschichten der Katalysator eingesetzt wird und hier somit eine beschleunigte Abbindung erfolgt, obwohl die Erwärmung der Mittelschicht zwangsläufig relativ verzögert von der Außenseite her erfolgt. Die hohe Temperatur, die vom Preßwerkzeug einwirkt, heizt zunächst die Deckschichten stark auf und bewirkt einen Dampfstoß, der die Temperatur (knapp über 100°C) ins Innere der Spanplatten überträgt. Bereits bei dieser Temperatur reagieren dann die katalysatorhaltigen Polyisocyanat-Bindemittel in der Mittelschicht in deutlich verkürzter Zeit ab. Eine weitere, wesentliche Verkürzung dieser Zeit ist kaum mehr realisierbar, da die Wärmeübertragung in die Mittelschicht nicht mehr schneller erfolgen kann. Es ist demgemäß bevorzugt, nur die Mittelschicht erfindungsgemäß zu aktivieren und die Deckschichten ohne Katalysator zu formulieren.

Man kann aber auch die Deckschicht-Beleimung der Späne mit aktivatorhaltigen Polyisocyanaten vornehmen, doch bringt dies keine wesentliche Änderung, da der für den Erfolg wesentliche Effekt die ausreichende Bindung in der Mittelschicht ist. In der Praxis verfolgt man die ausreichende Bindung der Späne in den Schichten durch Messung der Dicke bzw. Dickenzunahme der gebildeten Platten nach Verlassen der Presse.

Infolge ihrer hervorragenden mechanischen Eigenschaften eignen sich die erfindungsgemäß hergestellten Platten oder Formteile auf Basis vin lignocellulosehaltigen oder anderen organischen und/oder anorganischen Rohstoffen vor allem für eine Verwendung im Bauwesen. Um den Platten oder Formteilen die hierfür im allgemeinen erforderliche Beständigkeit gegen Pilzbefall, Insektenfraß oder Feuereinwirkung zu verleihen, kann man den Bindemitteln oder den Rohstoffen die handelsüblichen Additive, z.B. wäßrige Polyethylenemulsionen organische oder anorganische Schutzmittel, in reiner Form oder als Lösung in einer Menge von ca. 0,05 bis 30 Gew.-%, vorzugsweise 0,5 bis 20 Gew.-%, bezogen auf gesamtes Material, zusetzen. Als Lösungsmittel kommen in Frage: Wasser oder organische Lösungsmittel, z.B. Rückstandsöle aus der Erdölaufarbeitung, Chlorkohlenwasserstoffe u.a. Die Verleimungsqualität wird hierdurch im allgemeinen nicht beeinträchtigt. Im Gegensatz zu Phenol/Formaldehyd-Harz-verleimten Platten treten bei den erfindungsgemäß hergestellten Werkstoffen dabei vorteilhafterweise weder Salzausblühungen noch "Ausbluten" ein.

Bedingt durch die hohe Klebkraft der erfindungsgemäßen Bindemittel neigen die hiermit imprägnierten bzw. gebundenen Formkörper häufig dazu, an den Oberflächen der heißen Pressen oder Formen zu haften. Dies kann durch Trennmittel, welche man dem Bindemittel zusetzt, vermieden werden. Eine andere Lösung besteht darin, die Trennmittel in reiner Form oder als Lösung auf die mit den Preßlingen in Berührung kommenden metallischen Oberflächen oder die Formlingsoberfläche aufzubringen. Als äußere Trennmittel kommen hierbei alle bisher zu diesem Zweck vorgeschlagenen Substanzen in Frage. Bevorzugt sind jedoch gemäß DE-OS 2 325 926 Verbindungen, welche bei Isocyanaten eine Isocyanuratbildung katalysieren, beispielsweise Phenol-Mannichbasen, Derivate des Hexahydrotriazins oder Alkalisalze von Carbonsäuren und/oder Seifen, gegebenenfalls in Lösung wie z.B. wäßrigem Diethylenglykol. Ein weiterer Lösungsweg, die Haftung auszuschalten, besteht darin, eine Trennschicht zwischen Preßling und metallischer Pressenoberfläche anzuordnen, wobei die Trennschicht aus Bahnen, Folien oder Zerkleinerungsmaterial verschiedener Rohstoffe (z.B. Kunststoffe, Papier, Holz, Metall) bestehen kann. Wie schon mehrfach erwähnt, können mit den erfindungsgemäß zu verwendenden Isocyanat-Bindemitteln im Vergleich zu herkömmlichen Bindemitteln auf Basis von Phenol/Formaldehyd- oder Harnstoff/Formaldehyd-Harzen bei der Spanplattenherstellung wesentliche Verbesserungen, sowohl im Hinblick auf die mechanischen Eigenschaften als auch in verfahrenstechnischer Hinsicht, erzielt werden. So ist es im Falle von Holzspanplatten möglich, entweder bei gleicher Bindemittelmenge wie bei Phenol/Formaldehyd- bzw. Harnstoff/Formaldehyd-Harzen eine um bis zu 50 % erhöhte Biegesteifigkeit (neben einer Verbesserung anderer mechanischer Eigenschaften) oder aber bei einer um etwa 25 bis 70 % erniedrigten Bindemittelkonzentration ein gleiches mechanisches Eigenschaftsbild zu erreichen.

Die folgenden Beispiele erläutern die Erfindung. Wenn nicht anders vermerkt, sind Zahlenangaben als Gewichtsteile bzw. Gewichtsprozente zu verstehen.

### Beispiele

### Experimenteller Teil

### A) Latente Aktivatoren

1. 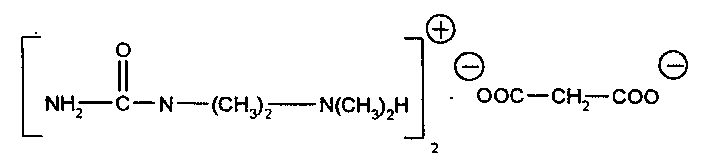 Aus 290 g (2,0 m) 3-N,N-Dimethylaminopropylharnstoff und 131,3 g Wasser, umgesetzt bei 15°C mit 104,1 g (1,0 m) Malonsäure.
   Säurezahl: 210
   Aminzahl: 210
2. Teilneutralisierte Form von 1.
   Aus 253,7 g (1,75 m) N,N-Dimethylaminopropylharnstoff und 119,2 g Wasser, umgesetzt bei 15°C mit 104,1 g (1,0 m) Malonsäure.
   Säurezahl: 235
   Aminzahl : 205
3. Teilneutralisierte Form von 1
   Aus 217,5 g (1,50 m) N,N-Dimethylaminopropylharnstoff und 107,1 g Wasser, umgesetzt bei 15°C mit 104,1 g (1,0 m) Malonsäure.
   Säurezahl: 261
   Aminzahl: 196
4. 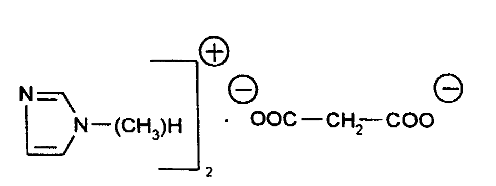 Aus 164,2 (2,0 m) N-Methylimidazol und 89,3 g Wasser, umgesetzt bei 15°C mit 104,1 g (1,0 m) Malonsäure.
   Säurezahl: 313
   Aminzahl: 313
5. 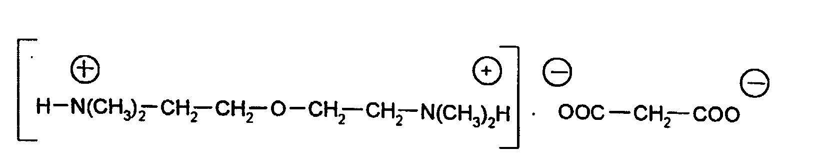 Aus 160 g (1,0 m) Bis-(2-N,N-Dimethylaminoethyl)-ether und 88,0 g Wasser, umgesetzt bei 15°C mit 104, g (1,0 m) Malonsäure.
   Säurezahl: 315
   Aminzahl: 314
6. 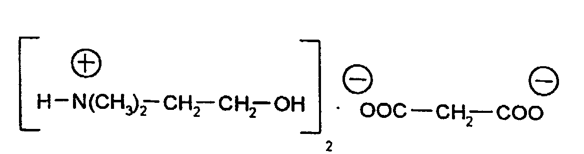 Aus 178 g (2,0 m) 2-N,N-Dimethylaminoethanol und 94 g Wasser, umgesetzt bei 15°C und 104,1 g (1,0 m) Malonsäure.
   Säurezahl: 296
   Aminzahl: 295
7. 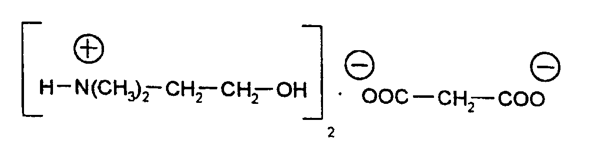 Aus 178 g (2,0 m) 2-N,N-Dimethylaminoethanol und 94 g N,N-Dimethylacetamid, umgesetzt bei 15°C mit 104,1 g (1,0 m) Malonsäure.
   Säurezahl: 295
   Aminzahl: 293
8. 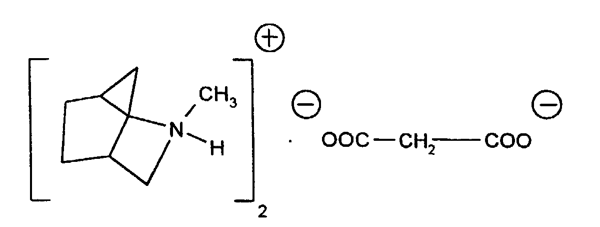 Aus 222 g (2,0 m) N-Methyl-2-azanorbonan und 108 g Wasser, umgesetzt bei 15°C mit 104,1 g (1,0 m) Malonsäure.
   Säurezahl: 258
   Aminzahl: 251
9. (Vergleich) 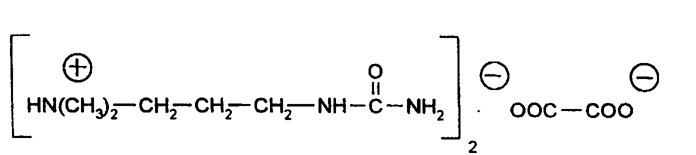 Aus 290 g (2,0 m) 3-N,NDimethylaminopropylharnstoff und 380,0 g Wasser, umgesetzt bei 15°C mit 90,1 g (1,0 m) Oxalsäure.
   Säurezahl: 146
   Aminzahl: 145
10. (Vergleich) 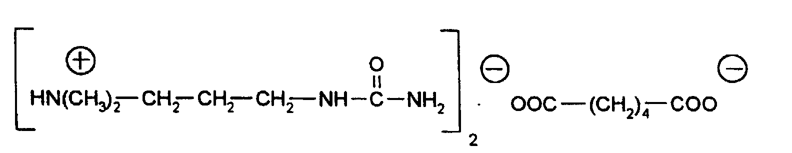 Aus 290 g (2,0 m) 3-N,N-Dimethylaminopropylharnstoff und 145,3 g Wasser, umgesetzt mit 146 g (1,0 m) Adipinsäure bei 15°C.
   Säurezahl: 191
   Aminzahl: 190

### Anwendungsbeispiel zur Herstellung einer 3-Schicht-Platte

A) Mittelschicht
2250 Gew.-Teile Mittschichtspäne, die aus einem Gemisch aus Nadelholz und Laubholz bestehen und einen Feuchtigkeitsgehalt von ca. 10 % aufweisen, werden mit 102 Gew.-Teilen Polyisocyanat Desmodur VPPU 1520A20L der Bayer AG beleimt. Die verwendete Menge der erfindungsgemäßen Katalysatoren ist in den Tabellen 1 und 2 zu entnehmen.
B) Unter- und Oberschicht
980 Gew.-Teile Späne mit einem Feuchtigkeitsgehalt von 15 % werden mit 43 Gew.-Teilen Polyisocyanat Desmodur VPPU 15020A20L der Bayer AG beleimt. Es wird eine 3-Schicht-Formling der Größe 580 x 520 mm einer Unter- und Oberschicht von 10 mm und einer Mittelschicht von 20 mm hergestellt und bei 180°C verpreßt. Die Zulagebleche wurden vorher mit dem Trennmittel Baysilon LAV der Bayer AG behandelt. Die V 100-Querzugsfestigkeit der erhaltenen Spanplatten (Dicke 16 mm) wurde nach DIN 68 763 nach verschiedenen Preßzeiten bestimmt.

**Tabelle 1**

| Katalysator-Lösung in der Mittelschicht | Preßzeit bei 180°C 1,6 min |
|---|---|
| ohne Katalysator | 0,09 MPa |
| 10 % Kat. | 0,16 MPa |
| 5 % Kat. | 0,15 MPa |
| 1 % Kat. | 0,15 MPa |

**Tabelle 2**

| Katalysator-Lösung in der Mittelschicht | Preßzeit bei 180°C | |
|---|---|---|
| | 1,85 min | 1,45 min |
| ohne Katalysator | 0,17 | * |
| 5 % Kat. gemäß Beispiel 6 | 0,21 | 0,18 MPa |
| 1 % Kat. gemäß Beispiel 6 | 0,16 | 0,15 MPa |

| | | |
|---|---|---|
| * nicht meßbar, brüchig | | |

**Tabelle 3**

| Ammoniumsalz von | Latenzwirkung bei 60 bis 80°C | zügige Abbindereaktion bei 100°C |
|---|---|---|
| Oxalsäure (gemäß Beisp. 9, Vergleich) | gut | schlecht |
| Malonsäure (gemäß Beisp. 1) | gut | gut |
| Adipinsäure (gemäß Beisp. 10; Vergleich) | schlecht | schlecht |
| Maleinsäure (Vergleich) | schlecht | schlecht |
| Methylphosphonsäure (Vergleich) gemäß EP 133 680 | nicht ausreichend | nicht ausreichend |

### Ergebnisse:

Durch den Zusatz wurden in analoger Weise wie in Tabelle 3 beschreiben, zusätzlich noch Vergleichsversuche mit Oxalsäure-, Adipinsäure- und Maleinsäureblockierten Aminen durchgeführt. Zum Vergleich mit dem Stand der Technik wurde noch durch einen Versuch mit einem wärmeaktivierbaren Aktivator auf der Basis von Ammoniaksalzen der Phosphonsäure gemäß EP 133 680 ergänzt.

Die herausragende Probe der Malonsäure-Ammoniumsalze zeigt sich in der für die Herstellung der Spanplatten geforderten Latenzphase bis 80°C und in der Katalyse der zügigen Abbindereaktion bei 100 bis 110°C Mittelschichttemperatur im Vergleich zu den in Tabelle 3 aufgeführten Ammoniumsalzen weiterer Säuren.

### Beispiel 11

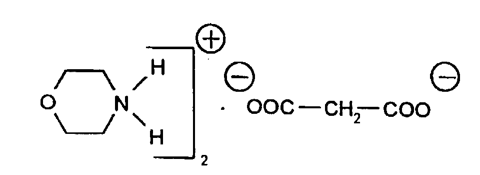

Aus 174 g (2,0 m) Morpholin und 92 g Wasser, umgesetzt bei 15°C und 104,1 g (1,0 m) Malonsäure.
Saure Zahl: 300
Aminzahl: 302

**Tabelle 4**

| Katalysator-Lösung in der Mittelschicht | Preßzeit bei 180°C | |
|---|---|---|
| | 1,85 min | 1,45 min |
| ohne Katalysator | 0,17 MPa | * |
| 1 % Kat. gemäß Beispiel 11 | 0,17 MPa | 0,16 MPa |

| | | |
|---|---|---|
| * nicht meßbar, brüchig | | |

Eine mechanisch hochwertige Verleimung wird auch in Beispiel 11 nach 145 min erreicht, entsprechend dem Ergebnis mit Beispiel 6.

### Latenzphase

Bis 80°C zeigt der aus den Holzspänen, Wasser, Katalysatorlösung 11 und Polyisocyanat bestehende Holzspanwerkstoff keine Reaktion zwischen Wasser ung Polyisocyanat. Diese wird erst - wie gewünscht - bei Temperaturen von 110°C in mittleren Spanschichten wie auch in Beispiel 6 bewirkt.

## Patentansprüche

1. Verfahren zur Herstellung von Preßwerkstoffen, insbesondere Holz-Spanplatten, durch Heißverpressen von mit Polyisocyanaten als Bindemittel vermischten und/oder imprägnierten lignosecellulosehaltigen Werkstoffen unter Verwendung von latenten durch Wärme aktivierbaren Katalysatoren, dadurch gekennzeichnet, daß als Katalysatoren Ammoniumsalze aus der Umsetzung von Aminen mit Malonsäure eingesetzt werden.

2. Verfahren gemäß Anspruch 1, dadurch gekennzeichnet, daß Ammoniumsalze aus der Umsetzung von tertiären Aminen mit Malonsäure eingesetzt werden.

3. Verfahren gemäß Anspruch 1 oder 2, dadurch gekennzeichnet, daß Ammoniumsalze aus der Umsetzung von N,N-Dimethylaminoethanol, Dimethylaminopropylharnstoff, Bis-2,2-Dimethylaminoethylether, N-Methylimidazol und/oder N-Methyl-2-azanorbonan mit Malonsäure eingesetzt werden.

4. Verfahren gemäß einem der Ansprüche 1 bis 3, dadurch gekennzeichnet, daß der Katalysator in einer Konzentration zwischen 0,1 bis 20 Gew.-%, vorzugsweise 0,1 bis 15 Gew.-%, bezogen auf das NCO-Gruppen aufweisende Bindemittel, eingesetzt wird.

5. Verfahren gemäß einem der Ansprüche 1 bis 4, dadurch gekennzeichnet, daß neben dem Polyisocyanat-Bindemittel Bindemittel auf Basis Harnstoff/Formaldehyd und/oder Melamin/Formaldehyd- und/oder Phenol/Formaldehy-Harzen mit eingesetzt und gegebenenfalls weitere Hilfs- und Zusatzmittel, Trennmittel, Holzschutzmittel, Flammschutzmittel oder Polyethylendispersionen zugesetzt werden.

6. Verfahren gemäß einem der Ansprüche 1 bis 5, dadurch gekennzeichnet, daß als Polyisocyanat polymeres 4,4-Methylendiisocyanat (PMDI) eingesetzt wird.

7. Verfahren gemäß einem der Ansprüche 1 bis 5, dadurch gekennzeichnet, daß als Polyisocyanat durch Polyetherpolyole oder Polyesterpolyole modifizierte PMDI-Typen eingesetzt werden.

## Claims

1. Process for the production of compression moulded materials, in particular chipboard, by hot pressing of materials containing lignocellulose mixed and/or impregnated with polyisocyanates as binders using latent, thermally activatable catalysts, characterised in that the catalysts used are ammonium salts from the reaction of amines with malonic acid.

2. Process according to claim 1, characterised in that ammonium salts from the reaction of tertiary amines with malonic acid are used.

3. Process according to claim 1 or 2, characterised in that ammonium salts from the reaction of N,N-dimethylaminoethanol, dimethylaminopropyl urea, bis-2,2-dimethylaminoethyl ether, N-methylimidazole and/or N-methyl-2-azanorbornane with malonic acid are used.

4. Process according to one of claims 1 to 3,
characterised in that the catalyst is used in a concentration of between 0.1 and 20 wt.%, preferably of 0.1 to 15 wt.%, relative to the binder containing NCO groups.

5. Process according to one of claims 1 to 4,
characterised in that, in addition to the polyisocyanate binder, binders based on urea/formaldehyde and/or melamine/formaldehyde and/or phenol/formaldehyde resins are also used and further auxiliary substances and additives, release agents, wood preservatives, flame retardants or polyethylene dispersions are optionally added.

6. Process according to one of claims 1 to 5,
characterised in that polymeric 4,4-methylene diisocyanate (PMDI) is used as polyisocyanate.

7. Process according to one of claims 1 to 5,
characterised in that PMDI grades modified by polyether polyols or polyester polyols are used as polyisocyanate.

## Revendications

1. Procédé de production de matériaux moulés par compression, de préférence des panneaux d'aggloméré en bois, par compression à chaud de matériaux contenant de la lignocellulose, mélangés à et/ou imprégnés de polyisocyanates comme liant, avec utilisation de catalyseurs latents, activables par la chaleur, caractérisé en ce que l'on met en oeuvre comme catalyseur, des sels d'ammonium provenant de la réaction d'amines avec l'acide malonique.

2. Procédé suivant la revendication 1, caractérisé en ce que l'on met en oeuvre des sels d'ammonium provenant de la réaction d'amines tertiaires avec l'acide malonique.

3. Procédé suivant la revendication 1 ou 2, caractérisé en ce que l'on met en oeuvre les sels d'ammonium provenant de la réaction du N,N-diméthylaminoéthanol, de la diméthylaminopropylurée, du bis-2,2-diméthylaminoéthyléther, du N-méthylimidazole et/ou du N-méthyl-2-azanorbornane avec l'acide malonique.

4. Procédé suivant l'une quelconque des revendications 1 à 3, caractérisé en ce que le catalyseur est mis en oeuvre en une concentration allant de 0,1 à 20% en poids, de préférence de 0,1 à 15% en poids, sur base du liant présentant des radicaux NCO.

5. Procédé suivant l'une quelconque des revendications 1 à 4, caractérisé en ce qu'en plus du liant polyisocyanate, on met en oeuvre un liant à base de résine urée/formaldéhyde et/ou mélamine/formaldéhyde et/ou phénol/formaldéhyde et facultativement, on ajoute d'autres additifs et auxiliaires, agent de séparation, agent de protection du bois, agent protecteur des flammes ou dispersions de polyéthylène.

6. Procédé suivant l'une quelconque des revendications 1 à 5, caractérisé en ce que l'on met en oeuvre, comme polyisocyanate, du 4,4-méthylénediisocyanate polymérique (PMDI).

7. Procédé suivant l'une quelconque des revendications 1 à 5, caractérisé en ce que l'on met en oeuvre, comme polyisocyanate, des types de PHDI modifiés par des polyéther-polyols ou des polyester-polyols.
